# EUROPEAN PATENT SPECIFICATION

(11) **EP 2 575 504 B1**
(45) Date of publication and mention of the grant of the patent: **05.12.2018**
(21) Application number: 11723737.0
(22) Date of filing: 25.05.2011
(51) Int. Cl.: A23L 33/00, A23L 33/135, A23L 33/21

(54) **IMMUNE IMPRINTING NUTRITIONAL COMPOSITION**
IMMUNSTEUERNDE ERNÄHRUNGSZUSAMMENSETZUNG
COMPOSITION NUTRITIONNELLE Ä EMPREINTE IMMUNITAIRE

(30) Priority: 25.05.2010 WO PCT/NL2010/050311
(43) Date of publication of application: 10.04.2013
(73) Proprietor: N.V. Nutricia, 2712 HM Zoetermeer (NL)
(72) Inventor: BEN AMOR, Kaouther, NL-3511 PH Utrecht (NL); KNIPPELS, Leon Matthieu Johannes, NL-3523 VJ Utrecht (NL); NAUTA, Alma Jildou, NL-3972 RD Driebergen (NL); GARSSEN, Johan, NL-3433 DA Nieuwegein (NL)
(74) Representative: Nederlandsch Octrooibureau
(86) International application number: PCT/NL2011/050356
(87) International publication number: WO 2011/149345

(56) References cited:
- EP-A1- 2 353 601
- WO-A1-2008/153377
- WO-A1-2009/072889
- WO-A1-2009/151315
- WO-A1-2010/130659
- WO-A1-2010/143961
- WO-A1-2011/110918
- WO-A2-2005/039319
- WO-A2-2006/091103
- WO-A2-2008/015374
- WO-A2-2008/153391
- HOUGEE ET AL.: "Oral treatment with probiotics reduces allergic symptoms in ovalbumin-sensitized mice: a bacterial strain comparative study", INT ARCH ALLERGY IMMUNOL, [Online] vol. 151, 15 September 2009 (2009-09-15), pages 107-117, XP002621455, DOI: doi=10.1159/000236000 Retrieved from the Internet: URL:http://content.karger.com/ProdukteDB/p rodukte.asp?doi=10.1159/000236000> [retrieved on 2011-02-09]
- KUITUNEN MIKAEL ET AL: "Probiotics prevent IgE-associated allergy until age 5 years in cesarean-delivered children but not in the total cohort", THE JOURNAL OF ALLERGY AND CLINICAL IMMUNOLOGY FEB 2009 LNKD- PUBMED:19135235,, vol. 123, no. 2, 15 September 2009 (2009-09-15), pages 335-341, XP002616479,
- TAYLOR ET AL: "Probiotic supplementation for the first 6 months of life fails to reduce the risk of atopic dermatitis and increases the risk of allergen sensitization in high-risk children: A randomized controlled trial", JOURNAL OF ALLERGY AND CLINICAL IMMUNOLOGY, ELSEVIER, AMSTERDAM, NL, vol. 119, no. 1, 1 January 2007 (2007-01-01), pages 184-191, XP022128225, ISSN: 0091-6749, DOI: 10.1016/J.JACI.2006.08.036
- SOH S E ET AL: "Probiotic supplementation in the first 6 months of life in at risk Asian infants -effects on eczema and atopic sensitization at the age of 1 year", CLINICAL & EXPERIMENTAL ALLERGY : JOURNAL OF THE BRITISH SOCIETY FOR ALLERGY AND CLINICAL IMMUNOLOGY, WILEY INTERSCIENCE, UK, vol. 39, no. 4, 1 April 2009 (2009-04-01), pages 571-578, XP002690481, ISSN: 0954-7894, DOI: 10.1111/J.1365-2222.2008.03133.X [retrieved on 2008-12-09]

## Description

### FIELD OF THE INVENTION

The invention relates to infant nutrition comprising probiotics which has long term effects on the immune system, in particular asthma, going beyond the time the composition has been administered and/or which prevents asthma medication.

### BACKGROUND OF THE INVENTION

Over the past decades, the prevalence of allergic diseases has risen in western countries. This increase has been hypothesized to result from diminished microbial exposure, leading to an altered composition of the intestinal microbiota. It has been shown that intestinal microbiota composition differs between children with and without atopy.

Several randomized controlled trials have been performed to investigate if intestinal microbiota manipulation with probiotics, living micro-organisms with immunomodulatory effects, reduce the severity of atopic dermatitis (AD). Children with AD have a chance of approximately 40% to develop asthma later in childhood, compared to 5-10% in the general population. Since AD is often the starting point of the so-called allergic march, probiotics may bring the atopic march to a halt in these children. However, so far human trials investigating prevention of allergic disease with probiotics in high risk children found no effect on the prevalence of asthma or asthma-like symptoms later in life and have a long term effect, exceeding the term the composition is administered.

Two prevention studies investigating the effect of probiotics in healthy infants at high risk for allergic disease did not show an effect on prevalence of recurrent wheeze at age 1-2 years (Taylor et al, 2007, J Allerg Clin Immunol 119: 184-191; Abrahamsson et al, 2007, J Allerg Clin Immunol 119: 1174-1180) and one study even showed an increased prevalence of recurrent (≥5) episodes of wheezing bronchitis in the probiotic group (Kopp et al, 2008, Pediatrics 121: e850-e.856). In two other prevention studies in high risk infants, one with pro- and one with synbiotics, asthma prevalence was determined at age 4-5 years and no difference between the treatment and placebo groups was found (Kalliomaki et al, 2003, Lancet 361: 1869-1871, Kuitinen et al, 2009, J Allerg Clin Immunol 123: 335-341). In Kukkonen et al, 2007 J Allergy Clin Immunol. 2007 Jan;119(1):192-8 at the age of 2 years no effects were observed on the total of allergic diseases, except in the subgroup of IgE positive infants, Arslanoglu et al, 2008, J. Nutr. 138:1091-1095, discloses the effect of administration early in life of a composition comprising prebiotics on cumulative incidence of recurrent wheezing up to the age of 2 years.

US 2006/233772 discloses the perinatal use of L GG for prevention of asthma in the off spring. Mouse experiments are disclosed.

WO 2008/153391 discloses the use of inactivated *B. breve* and a mix of non-digestible oligosaccharides for amongst others treatment and/or prevention of asthma.

WO 2006/091103 teaches the use of a composition comprising *B. breve* and non-digestible oligosaccharide for the treatment and/or prevention of gastro-intestinal, immune or endocrine disorders, like asthma, in an infant between 0-12 months.

### SUMMARY OF THE INVENTION

The inventors have surprisingly found that children, who had been administered a probiotic composition comprising *B. breve* during infancy, had a statistically significant lower prevalence of asthma-like symptoms and used less asthma medication when examined during a follow up later in life, than those children who had been administered a placebo during infancy. These results indicate that this specific probiotic composition has a preventive effect on development of asthma later in life.

### DETAILED DESCRIPTION OF THE INVENTION

The present invention thus concerns a nutritional composition comprising *Bifidobacterium breve* for administration to a human subject with an age from 0 to 6 months, for use in prevention of asthma or prevention of asthma-like symptoms selected from the group consisting of bronchial hyperresponsiveness, wheezing, noisy breathing and rattling breathing, when said human subject has reached an age above 16 months, and wherein said human subject to which the nutritional composition is to be administered suffers from dermatitis and/or eczema, wherein the nutritional composition further comprises protein selected form the group consisting of hydrolyzed milk protein and wherein the composition further comprises non-digestible oligosaccharides selected from the group consisting of fructo-oligosaccharides, galacto-oligosaccharides, gluco-oligosaccharides, arabino-oligosaccharides, mannan-oligosaccharides, xylo-oligosaccharides, fuco-oligosaccharides, arabinogalacto-oligosaccharides, glucomanno-oligosaccharides, galactomanno-oligosaccharides, sialic acid comprising oligosaccharides and uronic acid oligosaccharides.

### Bifidobacterium breve

The present composition comprises *Bifidobacterium breve. Bifidobacterium breve* is a Gram-positive, anaerobic, branched rod-shaped bacterium. The present *B. breve* preferably has at least 95 % identity of the 16 S rRNA sequence when compared to the type strain of *B. breve* ATCC 15700, more preferably at least 97% identity (Stackebrandt & Goebel, 1994, Int. J. Syst. Bacteriol. 44:846-849). Preferred *B. breve* strains are those isolated from the faeces of healthy human milk-fed infants. Typically, these are commercially available from producers of lactic acid bacteria, but they can also be directly isolated from faeces, identified, characterised and produced. According to a preferred embodiment, the present composition contains at least one *B. breve* selected from the group consisting of *B. breve* Bb-03 (Rhodia/Danisco), *B. breve* M-16V (Morinaga), *B. breve* R0070 (Institute Rosell, Lallemand), B. breve BR03 (Probiotical), B. breve BR92) (Cell Biotech), DSM 20091, LMG 11613, YIT4065, FERM BP-6223 and CNCM 1-2219. Most preferably, the *B. breve* is selected from the group consisting of *B. breve* M-16V and *B. breve* CNCM 1-2219.

The present composition preferably contains 10² to 10¹³ colony forming units (cfu) *B. breve* per gram dry weight of the present composition, preferably 10⁴ to 10¹², more preferably 10⁵ to 10¹⁰, most preferably from 10⁵ to 1x10⁹ cfu *B. breve* per gram dry weight of the present composition. The dose of *B. breve* according to the present invention is preferably administered at a daily dose of 10² to 10¹³, more preferably from 10⁵ to 10¹², most preferably from 10⁸ to 5x10¹⁰ colony forming units (cfu). Preferably the composition comprises 10³ to 10¹³ cfu *B. breve* per 100 ml, more preferably 10⁶ to 10¹¹ cfu *B. breve* per 100 ml, most preferably 10⁷ to 10¹⁰ cfu *B. breve* per 100 ml.

The present composition preferably comprises viable *B. breve.* Alternatively, the present composition preferably comprises non-viable *B. breve* equivalent to the amounts of CFU as described above. The equivalent of cfu can be determined by performing the 5'nuclease assay with the *B. breve* probes and primers as disclosed in WO 2005/039319 in the product (i.e. an infant formula) comprising non-viable *B. breve* and compare this with a calibration curve obtained from a comparable product (for instance a standard infant formula) to which known amounts in cfu of viable, preferably dried, *B. breve* cfu have been added. The dried viable bifidobacteria can be commercially obtained as described above. *B. breve* cells can be made non-viable by methods known in the art, including heat treatment steps (including sterilization, pasteurization, UHT treatment), radiation (UV), treatment with oxygen, treatment with bactericidals such as ethanol, sonication, ultra high pressure application, high pressure homogenization and use of a cell disruptor. Preferably the *B. breve* is heat-killed. The presence of non-viable *B. breve* advantageously provides many product technological benefits, including increased shelf-life, a reduced incidence of bacterial contamination, decreased post-acidification of the product, improved dosage control and improved convenience of reconstitution.

The *B. breve* of the present invention is preferably not genetically modified. Genetic modification is disadvantageous with respect to safety and consumer acceptance. Furthermore, genetic modification is costly and usually negatively affects strain growth properties. It is preferred to use

### Non-digestible oligosaccharides

Preferably the present composition comprises non-digestible oligosaccharides with a degree of polymerization (DP) between 2 and 250, more preferably 3 and 60. Non-digestible oligosaccharides further support the preventive effect on asthma later in life and on asthma medication. These effects are synergistic. The term "oligosaccharide" as used in the present invention preferably refers to a saccharide with a degree of polymerization (DP) of 2 to 250, preferably a DP 2 to 100, more preferably 2 to 60. It is understood that in the context of this invention a saccharide with a DP in a certain range may include a mixture of saccharides with different average DP's, for example, if an oligosaccharide with a DP of 2 to 100 is included in the present composition, this may include compositions which contain oligosaccharides with an average DP between 2 and 5, an average DP between 50 and 70 and an average DP between 7 and 60. The term "non-digestible oligosaccharide" as used in the present invention refers to oligosaccharides which are not or only partially digested in the intestine by the action of acids or digestive enzymes present in the human upper digestive tract (small intestine and stomach) but which are fermented by the human intestinal flora. For example, sucrose, lactose, maltose and maltodextrins are considered digestible. For example, galacto-oligosaccharides, fructo-oligosaccharides are considered non-digestible oligosaccharide.

The non-digestible oligosaccharide is selected from the group consisting of fructo-oligosaccharides (such as inulin), galacto-oligosaccharides (such as transgalacto-oligosaccharides or beta-galacto-oligosaccharides), gluco-oligosaccharides (such as gentio-, nigero- and cyclodextrin-oligosaccharides), arabino-oligosaccharides, mannan-oligosaccharides, xylo-oligosaccharides, fuco-oligosaccharides, arabinogalacto-oligosaccharides, glucomanno-oligosaccharides, galactomanno-oligosaccharides, sialic acid comprising oligosaccharides and uronic acid oligosaccharides. Preferably the composition comprises gum acacia on combination with a non-digestible oligosaccharide.

Preferably the present composition comprises fructo-oligosaccharides and/or galacto-oligosaccharides, more preferably galacto-oligosaccharides, most preferably transgalacto-oligosaccharides. In a preferred embodiment the composition comprises a mixture of transgalacto-oligosaccharides and fructo-oligosaccharides. Preferably the present composition comprises galacto-oligosaccharides with a DP of 2-10 , preferably with an average DP between 2 and 10, and/or fructo-oligosaccharides with a DP of 2-60, preferably with an average DP between 2 and 60, preferably with an average DP between 10 and 60, preferably with an average DP between 20 and 60. Preferably the composition comprises galacto-oligosaccharides and fructo-oligosaccharides in a weight ratio of 20 to 0.5, more preferably 20 to 1, most preferably from 12 to 2.

The galacto-oligosaccharide is preferably selected from the group consisting of transgalacto-oligosaccharides, lacto-N-tetraose (LNT), lacto-N-neotetraose (neo-LNT), fucosyl-lactose, fucosylated LNT and fucosylated neo-LNT. In a particularly preferred embodiment the present method comprises the administration of transgalacto-oligosaccharides ([galactose]ₙ-glucose; wherein n is an integer between 1 and 60, i.e. 2, 3, 4, 5, 6, ...., 59 ,60; preferably n is selected from 2, 3, 4, 5, 6, 7, 8, 9, or 10). Transgalacto-oligosaccharides (TOS) are for example sold under the trademark Vivinal™ (Borculo Domo Ingredients, Netherlands). Preferably the saccharides of the transgalacto-oligosaccharides are β-linked.

The present composition preferably contains fructooligosaccharide. The term "fructo-oligosaccharide" as used herein refers to a non-digestible polysaccharide comprising a chain of at least 2 β-linked fructose units, with a DP of 2 to 250, preferably 7 to 100, more preferably 20 to 60. Preferably inulin is used. Inulin is for example available under the tradename "Raftilin HP®", (Orafti). The average DP of the present fructo-oligosaccharide is preferably at least 7, more preferably at least 10, preferably below 100. The fructo-oligosaccharide used preferably has the (majority of) fructose units linked with a β(2→1) linkage. Other terms for fructooligosaccharides include inulin, fructopolysaccharide, polyfructose, fructans and oligofructose. The present composition preferably comprises fructo-oligosaccharides with a DP of 2 to 200.

Preferably, the composition comprises of 80 mg to 2 g non-digestible oligosaccharides per 100 ml, more preferably 150 mg to 1.50 g, even more preferably 300 mg to 1 g per 100 ml. Based on dry weight, the composition preferably comprises 0.25 wt.% to 20 wt.%, more preferably 0.5 wt.% to 10 wt.%, even more preferably 1.5 wt.% to 7.5 wt..% non-digestible oligosaccharides. A lower amount of non-digestible oligosaccharides will be less effective in preventing asthma later in life, whereas a too high amount will result in side-effects of bloating and abdominal discomfort.

Preferably the composition comprises 10² to 10¹³ cfu *B. breve* per gram and 0.25 wt.% to 20 wt.% non-digestible oligosaccharides based on dry weight, more preferably 10⁵ to 10¹⁰ cfu *B. breve* per gram and 0.5 wt.% to 10 wt.% non-digestible oligosaccharides based on dry weight.

Preferably the composition comprises 10³ to 10¹³ cfu *B. breve* and 80 mg to 2 g non-digestible oligosaccharides per 100 ml, more preferably 10⁶ to 10¹¹ cfu *B. breve* and 300 mg to 1 g non-digestible oligosaccharides per 100 ml.

Preferably the nutritional composition comprises i) 1x10⁵ cfu to 1x10¹⁰ cfu *B. breve* per g dry weight, more preferably 1x10⁶ cfu to 1x10¹⁰ cfu; and either ii) 0.5 to 20 wt.% galacto-oligosaccharides based on dry weight, more preferably 0.5 to 10 wt.% galacto-oligosaccharides or iii) 0.05 to 2 % fructo-oligosaccharides based on dry weight, more preferably 0.1 to 1 wt.% fructo-oligosaccharides or both ii) and iii).

### Compositions

The present composition is preferably enterally administered, more preferably orally.

The present composition is preferably a nutritional formula, preferably an infant formula. The present composition can advantageously be applied as a complete nutrition for infants. The present composition preferably comprises lipid, protein, and carbohydrate and is preferably administered in liquid form. The present invention includes dry compositions, e.g. powders, which are accompanied with instructions as to admix said dry compositions, in particular nutritional formula, with a suitable liquid, e.g. water.

The present invention advantageously concerns a composition wherein the lipid provides 5 to 50% of the total calories, the protein provides 5 to 50% of the total calories, and the carbohydrate provides 15 to 90% of the total calories. Preferably, in the present composition the lipid provides 35 to 50% of the total calories, the protein provides 7.5 to 12.5% of the total calories, and the carbohydrate provides 40 to 55% of the total calories. For calculation of the % of total calories for the protein component, the total of energy provided by the proteins, peptides and amino acids needs to be taken into account.

The present composition preferably comprises at least one lipid selected from the group consisting of animal lipid (excluding human lipids) and vegetable lipids. Preferably the present composition comprises a combination of vegetable lipids and at least one oil selected from the group consisting of fish oil, animal oil, algae oil, fungal oil, and bacterial oil. The present composition comprising non-digestible oligosaccharides excludes human milk. The protein component used in the nutritional preparation are preferably selected from the group consisting of non-human animal proteins (preferably milk proteins, preferably proteins from cow's milk), vegetable proteins (preferably soy protein and/or rice protein), free amino acids and mixtures thereof. The present composition preferably contains casein, whey, hydrolysed casein and/or hydrolysed whey protein. Preferably the protein comprises intact proteins, more preferably intact bovine whey proteins and/or intact bovine casein proteins. As the present composition is for use by infants suffering from allergy, the protein is preferably selected from the group consisting of hydrolyzed milk protein, more preferably hydrolyzed whey protein.

The liquid nutritional composition preferably has a caloric density between 0.1 and 2.5 kcal/ml, even more preferably a caloric density of between 0.5 and 1.5 kcal/ml, most preferably between 0.6 and 0.8 kcal/ml.

The amount of nutritional composition administered per day is preferably between 50 and 2000 ml, more preferably between 200 and 1500, most preferably between 400 and 1000 ml.

### Application

The infant and/or toddler nutrition according to the present invention has been found to be particularly useful as a nutrition for prematurely born babies, maturely born babies (vaginally as well as caesarean section delivered infants), infants which are in the adaptation period to solid food, infants and/or toddlers with an increased risk for or suffering from allergic eczema, from allergy, and/or infants and/or toddlers with an increased risk for infections, such as infants and/or toddlers attending day care centres, or suffering from infections. The invention is particularly advantageous for vaginally born infants. The invention is particularly advantageous for caesarean section delivered infants since these infants have an impaired microbial colonisation of the large intestine and an increased risk on development of asthma later in life. The invention is particularly advantageous for vaginally born infants. The invention is particularly advantageous for infants suffering from allergic eczema (also referred to as atopic dermatitis, atopic eczema or allergic dermatitis) since these infants have an increased risk on development of asthma later in life.

Herewith described is a method for providing nutrition to a human infant and/or toddler, said method comprising administering to the infant and/or toddler the present composition. The infant and/or toddler has an age between 0 and 6 months. The effect on asthma, asthma medication and asthma symptoms is when the human subject has reached an age above 16 months, more preferably above 24 months, more preferably above 36 months, more preferably above 5 years. It is noted that the phrase "between x and y months" means that the numbers x and y are included, e.g. between 0 and 12 months means from the first day a subject is born up to and including the last day of the 12^{th} month after birth of the subject, hence from 0 to 12 months. Thus "between x and y months" means "from x to y months".

Preferably the composition of the present invention is administered for a period of at least 4 weeks, more preferably at least 8 weeks, most preferably at least 12 weeks. A shorter period of administration will result in less effects later in life.

Particularly the present invention provides a composition as described herein above accompanied with indications (e.g. written material) comprising statement that the administration of the composition (e.g. to the infant) prevents asthma later in life and/or reduced the use and/or prevents asthma medication later in life.

Asthma is defined as a disorder in which chronic inflammation of the bronchial tubes (bronchi) makes the bronchial tubes swell and narrows the airways. Asthma attacks all age groups but often starts in childhood. It is a disease characterized by recurrent attacks of breathlessness and wheezing, which vary in severity and frequency from person to person. In an individual, they may occur from hour to hour and day to day. This condition is due to inflammation of the air passages in the lungs and affects the sensitivity of the nerve endings in the airways so they become easily irritated. In an attack, the lining of the passages swell causing the airways to narrow and reducing the flow of air in and out of the lungs.

Several predictive factors for the actual development of asthma have been identified. These include having AD, frequent wheezing and wheezing apart from colds. Since all children included in our study have AD and significant group differences in these specific predictive variables were found, the inventors believe that there will also be group differences in asthma prevalence later in life.

The present invention can also be used to prevent or reduce asthma medication, in particular later in life. Asthma medication can comprise the use of anti-inflammatory drugs and bronchodilators.

Anti-inflammatory drugs are the most important type of therapy for most people with asthma because these asthma medications prevent asthma attacks on an ongoing basis. Steroids, also called "cortisones" or "corticosteroids," are an important type of anti-inflammatory medication for people suffering from asthma. These asthma medications reduce swelling and mucus production in the airways. As a result, airways are less sensitive and less likely to react to triggers. Potential side effects include death when treatment is suddenly discontinued or tapered too quickly. Also the detection of infections may be delayed, which may be dangerous and reduces the ability to cope with trauma, surgery, and infection. Others side effects include acne, hairiness, stunted growth, increased appetite, weight gain, round face, abdominal pain, increased blood pressure, cataracts, dry mouth, bruising, fatigue, leg cramps, and increased perspiration, candidiasis (thrush), dysphonia, hoarseness, fluid retention, mood swings, increased cholesterol, osteoporosis, dermal thinning, diabetes, cataracts, and muscle weakness.

Bronchodilators relieve the symptoms of asthma by relaxing the muscle bands that tighten around the airways. This action rapidly opens the airways, letting more air come in and out of the lungs. As a result, breathing improves. Bronchodilators also help clear mucus from the lungs. As the airways open, the mucus moves more freely and can be coughed out more easily. It includes beta-antagonists, theophylinne and anticholinergicss. General potential side effects are nausea, vomiting, headaches, nervousness, restlessness, insomnia. Specific side effects of Beta-Agonists are jitters, tremors, flushing, headaches, rapid and/or irregular heart rate, overuse can cause air tubes to spasm. Specific side effects of theophylline are intestinal discomfort, nausea, vomiting, shakiness, diarrhea, headaches, insomnia, depression, increased and/or irregular heart rate, and leg cramps. Specific side effects of anticholinergics are headaches, dry mouth, coughing.

### Example 1

Ninety full-term infants, aged 0 to 7 months, fulfilling Hanifin and Rajka criteria for atopic dermatitis, were recruited. Inclusion criteria included a SCORing Atopic Dermatitis (SCORAD) score > 15, exclusively formula fed at time of enrolment, no other major medical problems and no use of probiotics or immunomodulatory medication during the 4 weeks before enrolment. Written informed consent was obtained from both parents of all participants.
Participants were randomized, to receive as test composition an extensively hydrolyzed whey based formula (Nutrilon Pepti®, Nutricia, Zoetermeer, the Netherlands) with additional synbiotics or as placebo the same formula without synbiotics for a period of 12 weeks. The investigators, participant's own physicians and parents were all blind to the treatment groups. One year after start of the intervention period participants returned for a follow-up visit, performed by the same investigator, who was still blinded to the treatment groups. During this visit parents were asked about respiratory symptoms (cough, shortness of breath, noisy/rattly breathing, wheezing) and medication use of their child, using a validated questionnaire. Synbiotics consisted of *Bifidobacterium breve* M16-V, 1.3 x 10⁹ cfu/100 ml and a mixture of 90% galacto-oligosaccharides (source Vivinal GOS, Borculo) and 10% fructo-oligosaccharides (source raftilin HP, Orafti), 0.8 g/100 ml. Children with an age below 6 month received starter formula. Children with an age of or above 6 month received follow on formula.), Formula was given on demand.

The primary outcome measure of this randomized controlled trial, were change in severity of atopic dermatitis after 12 weeks of intervention. Respiratory outcome measures at follow-up were: 1) prevalence of respiratory symptoms predictive of asthma: frequent wheezing, defined as ≥3 episodes after the intervention period, and wheezing apart from colds, 2) current use of asthma medication (beta-2 agonists, anticholinergics, inhaled corticosteroids).

Parametric data were analyzed with unpaired t-tests. Non-parametric data were analyzed with the Mann-Whitney U test. Binary data were analyzed using the χ²-test, or Fisher's exact test when appropriate, and results are represented as absolute risk reduction (ARR) with 95% confidence intervals (CI). SPSS software (15.0) was used for all analyses.

Ninety infants were randomized in the original study, the intention-to-treat consisted of 85 infants, of which 75 (88%) completed the one-year follow-up evaluation. Baseline characteristics of the children (gender, age, SCORAD index, Breast fed duration, parental asthma, parental smoking, pets, day care, older siblings, probiotics use after intervention period, use of asthma medication, cough, wheezing and noisy/rattling breathing were not statistically different between the two groups. The incidence and severance of atopic dermatitis as measured by SCORAD was not significantly different between the two groups at the end of the intervention at 12 weeks. Mean age at follow-up was 17.5 months (SD 1.6) in the test group and 17.2 (SD 1.8) in the placebo group.

The prevalence of asthma-like symptoms and use of asthma medication later in life in the test group and the placebo group are shown in table 1. Frequent wheezing (≥ 3 episodes after the intervention period), wheezing apart from colds and wheezing and/or noisy/rattly breathing apart from colds were significantly less prevalent in the test group than in the placebo group (ARR of wheezing without colds was significant, however the P value of the χ²-test was 0.056). Significantly fewer children in the test group than in the placebo group used asthma medication at time of follow-up. There were also significantly less new users of asthma medication (children that were using asthma medication at follow-up, but not at baseline) in the test group than in the placebo group.

In conclusion, it was demonstrated that infants with AD that received *Bifidobacterium breve* for a period of 12 weeks, have a lower prevalence of asthma-like symptoms and asthma medication use at one-year follow-up than those that received placebo. These results are indicative for a preventive effect on asthma-like symptoms and also on subsequent development of asthma.

**Table 1. Prevalence of asthma-like symptoms and asthma medication use at 1 year follow-up**

| | **Test comp.** n=36 n (%) | **Placebo** n =39 n (%) | **Difference** (ARR) (95% CI) % | ***P* value** |
|---|---|---|---|---|
| Frequent wheezing^{a} | 5 (13.9) | 13 (34.2) [n=38] | -20.3 (-39.2 to -1.5) | 0.04 |
| Wheezing apart from colds | 1 (2.8) | 7(17.9) | -15.2 (-28.4 to -2.0) | 0.056 |
| Wheezing and/or noisy breathing apart from colds | 1 (2.8) | 12 (30.8) | -28.0 (-43.4 to -12.5) | 0.001 |
| Asthma medication | 5 (13.9) | 13 (33.3) | -19.4 (-38.1 to -0.8) | 0.049 |
| Asthma medication at follow-up and not at baseline (new users) | 2 (5.6) | 10 (25.6) | -20.1 (-35.7 to - 4.5) | 0.02 |

| | | | | |
|---|---|---|---|---|
| ^{a}≥3 episodes after intervention period | | | | |

## Claims

1. A nutritional composition comprising viable *Bifidobacterium breve* for administration to a human subject with an age from 0 to 6 months, for use in prevention of asthma or prevention of asthma-like symptoms selected from the group consisting of bronchial hyperresponsiveness, wheezing, noisy breathing and rattling breathing, when said human subject has reached an age above 16 months, and wherein said human subject to which the nutritional composition is to be administered suffers from dermatitis and/or eczema, wherein the nutritional composition further comprises protein selected from the group consisting of hydrolyzed milk protein and wherein the composition further comprises non-digestible oligosaccharides selected from the group consisting of fructo-oligosaccharides, galacto-oligosaccharides, gluco-oligosaccharides, arabino-oligosaccharides, mannan-oligosaccharides, xylo-oligosaccharides, fuco-oligosaccharides, arabinogalacto-oligosaccharides, glucomanno-oligosaccharides, galactomanno-oligosaccharides, sialic acid comprising oligosaccharides and uronic acid oligosaccharides.

2. The nutritional composition for use according to claim 1, wherein the nutritional composition is administered for a period of at least 4 weeks, more preferably at least 8 weeks.

3. The nutritional composition for use according to any one of the preceding claims wherein the nutritional composition comprises at least 1x10⁵ cfu the *B. breve* per g dry weight.

4. The nutritional composition for use according to any one of the preceding claims wherein the *B. breve* is *B. breve* M16-V.

5. The nutritional composition for use according to any one of the preceding claims wherein the nutritional composition comprises galacto-oligosaccharides and/or fructo-oligosaccharides.

6. The nutritional composition for use according to any one of the preceding claims wherein the nutritional composition comprises more than 0.25 wt.% of non-digestible oligosaccharides based on dry weight.

7. The nutritional composition for use according to any one of the preceding claims, wherein the nutritional composition comprises
i. 1x10⁵ cfu to 1x10¹⁰ cfu *B. breve* per g dry weight, and either
ii. 0.5 to 20 wt.% galacto-oligosaccharides based on dry weight or
iii. 0.05 to 2 % fructo-oligosaccharides based on dry weight or both ii. and iii.

8. The nutritional composition for use according to any one of the preceding claims wherein the nutritional composition comprises lipid that provides 5 to 50% of the total calories, protein that provides 5 to 50% of the total calories, and carbohydrate that provides 15 to 90% of the total calories.

9. The nutritional composition for use according to any one of the preceding claims wherein the amount of composition to be administered per day is at least 50 ml.

## Patentansprüche

1. Nährstoffzusammensetzung, die lebensfähiges *Bifidobacterium breve* umfasst, zum Verabreichen an ein menschliches Subjekt im Alter von 0 bis 6 Monaten, zur Verwendung in der Prävention von Asthma oder der Prävention von asthmaartigen Symptomen, ausgewählt aus der Gruppe, bestehend aus bronchialer Überempfindlichkeit, Keuchen, geräuschvoller Atmung und rasselnder Atmung, wenn das besagte menschliche Subjekt ein Alter von mehr als 16 Monaten erreicht hat, und wobei das besagte menschliche Subjekt, dem die Nährstoffzusammensetzung verabreicht werden soll, an Dermatitis und/oder Ekzemen leidet, wobei die Nährstoffzusammensetzung ferner Protein umfasst, ausgewählt aus der Gruppe, bestehend aus hydrolysiertem Milchprotein und wobei die Zusammensetzung ferner nicht verdauliche Oligosaccharide umfasst, ausgewählt aus der Gruppe, bestehend aus Fructo-Oligosacchariden, Galacto-Oligosacchariden, Gluco-Oligosacchariden, Arabino-Oligosacchariden, Mannan-Oligosacchariden, Xylo-Oligosacchariden, Fuco-Oligosacchariden, Arabinogalacto-Oligosacchariden, Glucomanno-Oligosacchariden, Galactomanno-Oligosacchariden, Sialinsäure, die Oligosaccharide enthält, und Uronsäure-Oligosacchariden.

2. Nährstoffzusammensetzung zur Verwendung nach Anspruch 1, wobei die Nährstoffzusammensetzung für eine Dauer von zumindest 4 Wochen, darüber hinaus bevorzugt zumindest 8 Wochen, verabreicht wird.

3. Nährstoffzusammensetzung zur Verwendung nach einem der vorangehenden Ansprüche, wobei die Nährstoffzusammensetzung zumindest 1x10⁵ cfu des *B. breve* pro g Trockengewicht enthält.

4. Nährstoffzusammensetzung zur Verwendung nach einem der vorangehenden Ansprüche, wobei das *B. breve B. breve* M16-V ist.

5. Nährstoffzusammensetzung zur Verwendung nach einem der vorangehenden Ansprüche, wobei die Nährstoffzusammensetzung Galacto-Oligosaccharide und/oder Fructo-Oligosaccharide umfasst.

6. Nährstoffzusammensetzung zur Verwendung nach einem der vorangehenden Ansprüche, wobei die Nährstoffzusammensetzung mehr als 0,25 Gew.-% nicht verdaulicher Oligosaccharide, bezogen auf das Trockengewicht, umfasst.

7. Nährstoffzusammensetzung zur Verwendung nach einem der vorangehenden Ansprüche, wobei die Nährstoffzusammensetzung umfasst
i. 1x10⁵ cfu bis 1x10¹⁰ cfu *B. breve* pro g Trockengewicht, und entweder
ii. 0,5 bis 20 Gew.-% Galacto-Oligosaccharide, bezogen auf das Trockengewicht, oder
iii. 0,05 bis 2 % Fructo-Oligosaccharide, bezogen auf das Trockengewicht oder sowohl ii. als auch iii.

8. Nährstoffzusammensetzung zur Verwendung nach einem der vorangehenden Ansprüche, wobei die Nährstoffzusammensetzung Lipid, das 5 bis 50% der Gesamtkalorien bereitstellt, Protein, das 5 bis 50% der Gesamtkalorien bereitstellt, und Kohlenhydrat, das 15 bis 90% der Gesamtkalorien bereitstellt, umfasst.

9. Nährstoffzusammensetzung zur Verwendung nach einem der vorangehenden Ansprüche, wobei die Menge der Zusammensetzung, die pro Tag verabreicht wird, zumindest 50 ml ist.

## Revendications

1. Composition nutritionnelle comprenant de la *Bifidobacterium breve* viable pour administration à un sujet humain âgé de 0 à 6 mois, à utiliser dans la prévention de l'asthme ou la prévention des symptômes asthmatiformes choisis dans le groupe constitué par une hyperréactivité bronchique, un sifflement, une respiration bruyante et une respiration haletante, lorsque ledit sujet humain est âgé de plus de 16 mois, et dans laquelle ledit sujet humain auquel la composition nutritionnelle doit être administrée souffre de dermatite et/ou d'eczéma, dans laquelle la composition nutritionnelle comprend en outre une protéine sélectionnée dans le groupe comprenant une protéine de lait hydrolysée, et dans laquelle la composition comprend en outre des oligosaccharides non digestibles choisis dans le groupe constitué des fructo-oligosaccharides, des galacto-oligosaccharides, des gluco-oligosaccharides, des arabino-oligosaccharides, des mannan-oligosaccharides, les xylo-oligosaccharides, des fuco-oligosaccharides, des arabinogalacto-oligosaccharides, des glucomanno-oligosaccharides, des galactomanno-oligosaccharides, de l'acide sialique comprenant des oligosaccharides et des oligosaccharides d'acide uronique.

2. Composition nutritionnelle à utiliser selon la revendication 1, dans laquelle la composition nutritionnelle est administrée pendant une période d'au moins 4 semaines, de manière plus préférée d'au moins 8 semaines.

3. Composition nutritionnelle à utiliser selon l'une quelconque des revendications précédentes, dans laquelle la composition nutritionnelle comprend au moins 1 x 10⁵ cfu de *B. breve* par gramme de poids sec.

4. Composition nutritionnelle à utiliser selon l'une quelconque des revendications précédentes, dans laquelle la *B. breve* est *B. breve* M16-V.

5. Composition nutritionnelle à utiliser selon l'une quelconque des revendications précédentes, dans laquelle la composition nutritionnelle comprend des galacto-oligosaccharides et/ou des fructo-oligosaccharides.

6. Composition nutritionnelle à utiliser selon l'une quelconque des revendications précédentes, dans laquelle la composition nutritionnelle comprend plus de 0,25 % en poids d'oligosaccharides non digestibles sur la base du poids sec.

7. Composition nutritionnelle à utiliser selon l'une quelconque des revendications précédentes, dans laquelle la composition nutritionnelle comprend
i. 1x10⁵ cfu à 1x10¹⁰ cfu de *B. breve* par gramme de poids sec, et soit
ii. 0,5 à 20 % en poids de galacto-oligosaccharides sur la base du poids sec, ou
iii. 0,05 à 2 % de fructo-oligosaccharides sur la base du poids sec, ou à la fois ii. et iii.

8. Composition nutritionnelle à utiliser selon l'une quelconque des revendications précédentes, dans laquelle la composition nutritionnelle comprend un lipide qui fournit 5 à 50 % des calories totales, une protéine qui fournit 5 à 50 % des calories totales et un hydrate de carbone qui fournit 15 à 90 % du total des calories.

9. Composition nutritionnelle à utiliser selon l'une quelconque des revendications précédentes, dans laquelle la quantité de composition à administrer par jour est d'au moins 50 ml.
